Europäisches Patentamt

European Patent Office

Office européen des brevets

(11) Numéro de publication: **0 206 971**

**A1**

## (12) DEMANDE DE BREVET EUROPEEN

(21) Numéro de dépôt: 86630086.6

(22) Date de dépôt: 06.05.86

(51) Int. Cl.⁴: **A61M 5/24**

(30) Priorité: **29.05.85 LU 85916**

(43) Date de publication de la demande:
**30.12.86 Bulletin 86/52**

(84) Etats contractants désignés:
**AT BE CH DE FR GB IT LI LU NL SE**

(71) Demandeur: **VECTA GLASS COMPANY Ltd.
One Dunraven Street
London W1Y 3FG(GB)**

(72) Inventeur: **L'inventeur a renoncé àsa
désignation**

(74) Mandataire: **Waxweiler, Jean et al
OFFICE DENNEMEYER S.à.r.l. 21-25 Allée
Scheffer P.O.Box 41
L-2010 Luxembourg(LU)**

(54) **Seringue pour prompt emploi.**

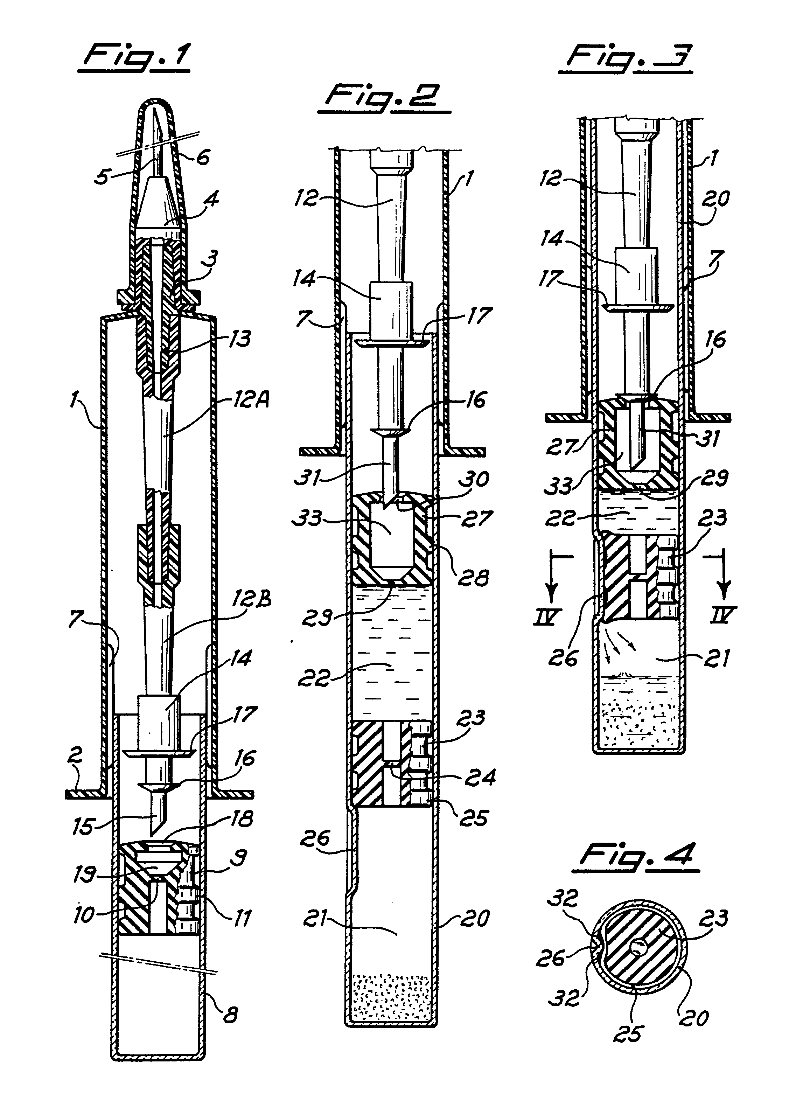

(57) L'invention concerne une seringue pour prompt emploi d'usage facile, de coût bas et de conditionnement rapide, ayant une sécurité élevée contre la pollution bactérienne, comprenant un container (8) de forme cylindrique pour la solution injectable ayant aussi la fonction de piston et fermé par un bouchon à piston (9), qui peut être perforé par une pointe - (15) sortant en bas d'un petit tube d'aspiration (12), fixé à l'enveloppe extérieure de la seringue en correspondance de la tête de raccord au cône d'attaque de l'aiguille d'administration. Une forme de réalisation de la seringue est montrée dans la Fig. 1.

Fig.1

## "SERINGUE POUR PROMPT EMPLOI"

La présente invention concerne une seringue pour prompt emploi, qui présente de nombreux avantages par rapport aux seringues pour prompt emploi actuellement existantes, qui ont une structure complexe et un coût de production élevé.

Par le terme seringue pour prompt emploi on entend une seringue dans laquelle on a déjà introduit, en phase de conditionnement, la solution injectable, et qui peut être préférablement contenue dans un étui protecteur stérile.

La seringue pour prompt emploi, selon la présente invention, n'élimine pas seulement les inconvénients susmentionnés, résultant ainsi de structure simplifiée, facile à employer et de coût bas, mais réduit en outre énormément la possibilité de pollution bactérienne de la solution injectable, du moment que cette dernière est en contact seulement avec un bouchon à piston de matériel stérile, à part naturellement les parois du container stérilisé en verre.

La seringue pour prompt emploi selon la présente invention est caractérisé par le fait de comprendre, en combinaison, un container de forme cylindrique pour la solution injectable ayant aussi la fonction de piston et fermé par un bouchon à piston, qui peut être perforé par une pointe sortant en bas d'un petit tube d'aspiration, fixé à l'enveloppe extérieure de la seringue en correspondance de la tête de raccord au cône d'attaque de l'aiguille d'administration.

Dans une variante de la présente invention, le container peut être utilisé comme ampoule de prompt emploi pour médicaments à deux composants à mélanger au moment de l'emploi. Dans ce cas, le container présente un deuxième bouchon intérieur qui divise le container en deux chambres, contenant chacune un composant, qui sont mises en contact entre elles, au moment de l'emploi, portant ce bouchon intérieur à se trouver en correspondance d'une saillie intérieure de la paroi du container qui déforme le bouchon intérieur, créant un passage de communication entre les deux chambres du container qui permet donc le mélange des composants.

D'ultérieures caractéristiques de la seringue, selon la présente invention, consistent dans des organes d'accrochage du petit tube d'aspiration au bouchon à piston avec possibilité de mouvement pour le contrôle de l'exécution correcte de la piqûre, dans un container approprié pour prélèvements de fluides corporels pour analyses, ainsi que dans la prévision d'organes intérieurs pour guider le glissement du container pour la solution injectable dans l'enveloppe extérieure de la seringue.

Ces buts-ci et d'autres, caractéristiques et avantages de la seringue pour prompt emploi, selon la présente invention, apparaissent d'ailleurs clairs et évidents par la description détaillée suivante de quelques formes de réalisation préférées, qui sont toutefois reportées ici à seul titre d'exemple et non limitatif de la portée de la découverte, faisant référence aux figures des tables de dessins illustratifs en annexe, dans lesquelles:

la Figure 1 est une vue totale sectionnée de la seringue pour prompt emploi selon la présente invention dans sa forme de réalisation plus générale pour médicaments à un seul composant;

la Figure 2 est une vue analogue partielle de la forme de réalisation pour médicament à deux composants, avant l'emploi;

la Figure 3 est une vue analogue à la Fig.2, mais montrant la phase de mélange des deux composants;

la Figure 4 est une section transversale exécutée le long de la ligne IV-IV de la Fig. 3, montrant les canalicules à travers lesquels a lieu le passage de composant de la chambre supérieure à la chambre inférieure;

la Figure 5 est une vue analogue aux Fig.2 et 3 main montrant la phase finale de préparation à la piqûre;

la Figure 6 est une vue analogue aux précédentes, montrant une forme de réalisation différente du bouchon extérieur du container pour le médicament à deux composants; et

la Figure 7 est une vue totale partiellement sectionnée d'une autre forme de réalisation de container, particulièrement étudié pour prélèvements de fluides corporels pour analyses, à employer avec la seringue pour prompt emploi selon la présente invention.

Faisant maintenant référence tout d'abord à la Fig. 1 des dessins annexés, on décrit la structure totale de la seringue pour prompt emploi selon la présente invention dans sa forme plus générale, c'est-à-dire celle avec ampoule pour médicament à un seul composant. La seringue présente donc une enveloppe extérieure 1, qui termine en bas par le bord habituel annulaire saillant 2 et en haut par une tête 3 de raccord au cône 4 d'attaque de l'aiguille d'administration 5, protégée par le capuchon protège-aiguille 6 relatif, le tout réalisé en matériel plastique approprié.

Dans le voisinage de l'embouchure inférieure ouverte, la surface intérieure de l'enveloppe extérieure 1 présente des saillies 7 qui servent de guide pour le container cylindrique ou ampoule 8 pour la solution injectable, généralement réalisé en verre, qui est fermé par un bouchon 9, de préférence en caoutchouc F.U. ou autre matériel approprié, lequel présente une cloison centrale 10 perforable au moment de l'emploi, et des protubérances annulaires extérieures 11 pour la parfaite étanchéité contre la paroi intérieure du container 8. Bien que la position des protubérances annulaires d'étanchéité 11 sur la surface extérieure du bouchon 9,ainsi que la position de la cloison ou membrane centrale 10 pourraient .aussi être différentes de celles montrées dans le dessin, la position asymétrique de celles-ci a été choisie pour faciliter le démoulage du bouchon de l'étampe, permettant donc une rapidité d'étampage sensiblement plus élevée et par conséquent une économie de production supérieure de la pièce.

Pour le prélèvement du liquide de l'intérieur du container ou ampoule 8 et son envoi à l'aiguille d'administration 5, on a prévu un petit tube d'aspiration composé par une certain nombre d'éléments modulaires 12 légèrement contracturés, qui se greffent l'un dans l'autre. Le module supérieur 12A se greffe sur une tige 13 faisant partie de la tête 3 du corps 1 de la seringue, tandis que le module inférieur 12B se greffe dans la base 14 d'une pointe perforante 15, dotée d'un petit collier de battue 16 et d'un anneau d'arrêt 17. L'anneau réalise une étanchéité avec le container en verre de manière à garantir une protection de la partie extérieure du piston et la pointe du perforateur qui doivent rester stériles. On peut aussi prévoir plusieurs anneaux d'étanchéité. Le petit tube d'aspiration peut aussi être constitué par un seul élément de longueur appropriée.

Quand on doit employer la seringue, en poussant en haut le container cylindrique ou ampoule 8, qui agit ainsi aussi en piston de la seringue, la pointe 15 passera à travers le trou supérieur ouvert 18 du bouchon 9 et perforera la cloison centrale 10, et ne pourra plus se détacher du bouchon 9,du moment que le collier de battue 16, qui se trouvera maintenant dans la cavité 19 formée dans le bouchon 9 entre cloison .centrale 10 et trou supérieur 18, ne peut plus sortir de ce dernier vu sa forme troncoconique qui en permet l'entrée mais pas la sortie. Toutefois la cavité 19 permet à la pointe 15 d'accomplir un mouvement limité déterminé par l'ampleur de la cavité 19 et par la distance entre collier de battue 16 et anneau d'étanchéité 17, qui consent d'effectuer le contrôle visuel habituel, quand on pratique la piqûre, pour vérifier au moyen d'une légère aspiration si l'aiguille a été introduite correctement dans les tissus corporels du patient.

Faisant maintenant référence aux Fig. 2-5, on illustre dans celles-ci la forme de réalisation de la seringue selon la présente invention, relative à l'ampoule pour médicaments à deux composants, qui peuvent être indifféremment une poudre médicamenteuse à dissoudre au moment de l'emploi avec le liquide solvant, ou deux liquides à mélanger seulement au moment de l'emploi.

Par conséquent, dans ce cas, l'ampoule ou container cylindrique 20 est subdivisé en deux chambres, la chambre inférieure 21 contenant la poudre médicamenteuse ou le premier liquide et la chambre supérieure 22 contenant le solvant ou le deuxième liquide. La chambre inférieure 21 est fermée par un bouchon 23 doté d'une cloison centrale 24 et d'anneaux extérieurs d'étanchéité 25, placé immédiatement au-dessus d'une protubérance intérieure ou empreinte 26, obtenue dans la paroi de verre de l'ampoule de l'extérieur vers l'intérieur, tandis que la chambre supérieure 22 est fermée par un bouchon 17 doté lui aussi d'anneaux extérieurs d'étanchéité 28, mais dans lequel la cloison perforable 29 coïncide avec le fond du bouchon, dont le sommet présente un trou d'embouchure ouvert 30.

Dans ce cas la pointe perforante 31 est sensiblement plus longue que la pointe 15 de la forme de réalisation de la Fig. 1, de même que la distance entre collier de battue 16 et anneau d'arrêt 17 est supérieure, pour les raisons qui apparaissent claires par la suite. On doit aussi mettre en évidence le fait que le petit tube d'aspiration peut être composé par plus de deux éléments modulaires 12, selon la longeur de la seringue, déterminée comme d'habitude par le volume de la solution à injecter (1cc, 2cc, 5cc, 10cc, etc.).

Dans la Fig. 3 on voit comme, en poussant vers le haut le container ou ampoule 20, le collier de battue 16 va s'appuyer sur le sommet du bouchon supérieur 27 qui est donc poussé en bas, agissant comme un piston et exerçant une pression sur le liquide contenu dans la chambre supérieure 22, pression qui est transférée sur le bouchon inférieur 23, qui est donc obligé de descendre jusqu'à s'encastrer contre l'empreinte ou protubérance 26, laquelle, écrasant le bouchon 23, ouvre une communication entre les deux chambres à travers les canalicules 32 qui se forment aux deux côtés de la protubérance 26, c'est pourquoi le liquide contenu dans la chambre supérieure 22 se transfère dans la chambre inférieure 21 et se mélange avec la poudre à solubiliser ou le deuxième liquide qui y est contenu, tandis que l'air qui se trouve dans la chambre inférieure 21 se transfère au-dessus du bouchon inférieur 23, et cette opération peut être effectuée en une ou plusieurs fois.

Après avoir terminé le mélange et en continuant encore à pousser l'ampoule ou container 20, on arrive à la position finale illustrée dans la Fig.5, où la pointe perforante 31 perce d'abord la cloison de fond 29 du bouchon supérieur 27, qui va s'arrêter contre le bouchon inférieur 23, par conséquent la pointe perforante 31 pénètre dans ce dernier et perce aussi sa cloison centrale 24, c'est pourquoi la solution peut être maintenant prélevée pour pratiquer la piqûre, et ici aussi la cavité intérieure 33 du bouchon supérieur 27 permet de retirer légèrement l'aiguille pour effectuer le contrôle de correcte introduction dans le corps du patient, sans que la pointe perforante 31 puisse se détacher, car empêchée par le collier de battue 16 comme dans la forme de réalisation de la Figure 1. Le même résultat pourrait de toute façon être obtenu avec un bouchon supérieur 34 légèrement différent, montré dans la Fig. 6, où sa cavité intérieure 35 est en forme de tronc de cône ouvert en haut: même dans ce cas le collier de battue 16 empêcherait la sortie de la pointe perforante 31, étant considérablement plus grand que l'embouchure de la cavité in térieure 35 et en outre évasé dans la direction opposée.

Il faut remarquer que dans les seringues pour prompt emploi actuellement existantes, quand on prévoit une ampoule bistade pour deux composants, elle est dotée d'une protubérance ou empreinte sortant vers l'extérieur, ce qui présente de considérables inconvénients aussi bien en phase de production que d'emploi. La solution de la présente invention,avec la protubérance ou empreinte saillant à l'intérieur de l'ampoule, permet au contraire de traiter le container à la manière d'un corps cylindriques sans protubérances extérieures qui en limitent les possibilités de travail sur des machines automatiques et ne permettent pas son étiquetage ou étampage aisé, et ainsi les temps et coûts de production,même de cet élément de la seringue,baissent considérablement. On doit aussi faire remarquer que dans cette ampoule ou container pour deux composants, la poudre à solubiliser peut aussi être obtenue en lyophilisant directement dans le container le médicament, précédemment introduit en solution dosée.

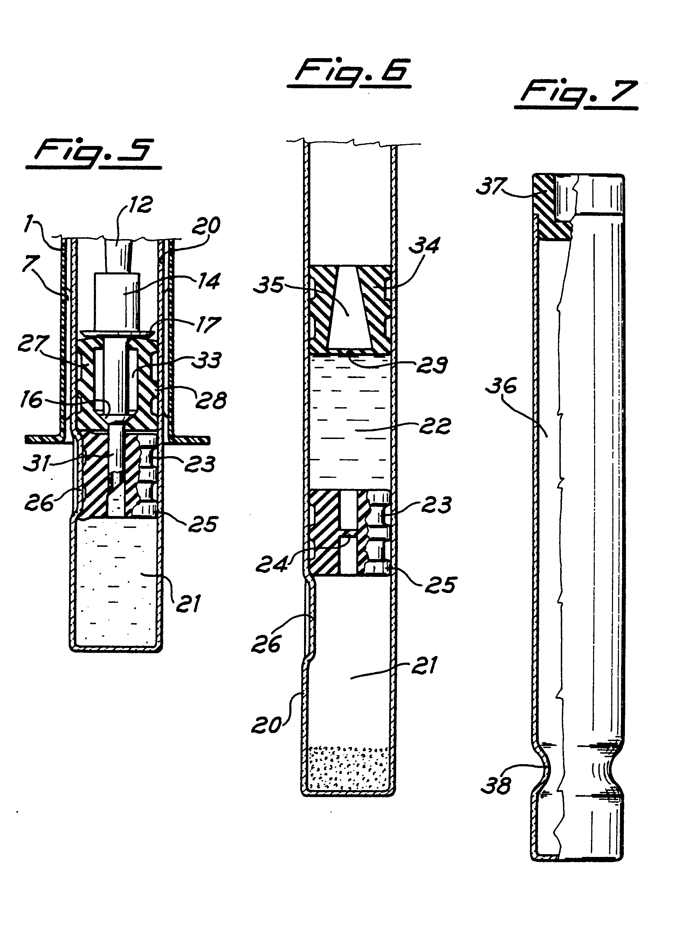

Enfin, dans la Fig. 7 on montre un container cylindrique 36 à employer comme piston avec la seringue de la présente invention, quand on doit effectuer des prélèvements de fluides corporels pour analyses, et dans ce cas le container est simplement doté d'un petit bouchon de fermeture 37 et de l'écrasement inférieur annulaire 38 pour sa manoeuvre aisée.

D'après tout cela il résulte donc évident que la seringue pour prompt emploi, selon la présente invention, réalise pleinement les buts susmentionnés, et on doit en outre comprendre que de nombreuses variantes, modifications, adjonctions et/ou substitutions pourront être apportées à cette dernière, sans pour autant s'éloigner de l'esprit et du but de la découverte, ni même de son cadre de protection, comme cela est d'ailleurs défini dans les revendications annexées.

## Revendications

1. Seringue pour prompt emploi, caractérisée par le fait de comprendre, en combinaison, un container de forme cylindrique pour la solution injectable ayant aussi la fonction de piston et fermé par un bouchon à piston, qui peut être perforé par une pointe sortant en bas d'un petit tube d'aspiration, fixé à l'eveloppe extérieure de la seringue en correspondance de la tête de raccord au cône d'attaque de l'aiguille d'administration.

2. Seringue pour prompt emploi selon la revendication 1, caractérisée par le fait que le petit tube d'aspiration est constitué par des éléments modulaires dotés de trou passant central, pouvant être greffés l'un dans l'autre dans un nombre déterminé par la longueur du corps de la seringue, à l'élément plus en bas vu que la pointe apte à perforer le bouchon à piston est greffée.

3. Seringue pour prompt emploi selon la revendication 1, caractérisée par le fait que le petit tube d'aspiration peut aussi être constitué par un seul élément de longueur adéquate.

4. Seringue pour prompt emploi selon la revendication 1, caractérisée par le fait que le bouchon à piston du container pour la solution injectable présente des protubérances annulaires extérieures d'étanchéité et guide sur la paroi intérieure de ce container.

5. Seringue pour prompt emploi selon la revendication 4, caractérisé par le fait que le bouchon à piston est percé centralement en correspondance d'une cloison ou tête de fer meture.

6. Seringue pour prompt emploi selon la revendication 1, caractérisée par le fait que la pointe perforant le bouchon à piston est dotée d'engins pour accrocher le piston même, empêchant le détachement réciproque au moment de l'emploi, et consentant le contrôle visuel,au moyen d'une légère aspiration,de la juste introduction de l'aiguille dans les tissus du patient.

7. Seringue pour prompt emploi selon la revendication 6, carractérisée par le fait que ces engins prévus sur la pointe perforante pour accrocher le piston sont constitués par un collier de battue en forme de tournette troncoconique, qui s'introduit dans une cavité intérieure du bouchon à piston, mais ne peut ensuite être extraite de son embouchure, consentant seulement un mouvement égal environ à la longueur de cette cavité.

8. Seringue pour prompt emploi selon la revendication 1, caractérisée par le fait que le corps ou enveloppe extérieure de la seringue présente sur sa paroi intérieure des saillies de guide pour le glissement dans celle-ci du container ou ampoule pour la solution injectable.

9. Seringue pour prompt emploi selon les revendications précédentes, caractérisée par le fait que pour l'utilisation comme ampoule pour médicaments à deux composants à mélanger au moment de l'emploi, le container cylindrique présente un deuxième bouchon intérieur qui divise le container en deux chambres, chacune contenant un composant, qui sont mises en contact entre elles au moment de l'emploi, portant ce bouchon intérieur à se trouver en correspondance d'une saillie ou empreinte intérieure de la paroi du container, qui déforme ce bouchon intérieur créant un passage de communication entre les deux chambres du container permettant donc le mélange des composants susdits.

10. Seringue pour prompt emploi selon la revendication 9, caractérisée par le fait que la pointe perforante du petit tube modulaire d'aspiration, après le mélange des deux composants, est apte à perforer les deux bouchons maintenant en contact entre eux, pour prélever la solution prête pour l'emploi, le mouvement de ces bouchons dans le container ou ampoule étant réalisé au moyen de la pression exercée par le collier de battue du petit tube modulaire, au fur et à mesure que l'on pousse en haut le container ou ampoule dans la seringue.

11. Seringue pour promt emploi selon les revendications précédentes, caractérisée par le fait de présenter aussi un container cylindrique apte à effectuer des prélèvements de fluides corporels, doté de bouchon de fermeture et de façonnage pour sa manoeuvre.

0 206 971

Fig.1

Fig.2

Fig.3

Fig.4

_Fig.5_

_Fig.6_

_Fig.7_

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-3 994 296  (H.S. CLOYD) <br> * Colonne  1, ligne 26 - colonne 2, ligne 68; figures 1-3 * | 1,3-6 | A 61 M   5/24 |
| | --- | | |
| Y | US-A-2 576 951  (LOCKHART) <br> * 'Description'; figures * | 1,9 | |
| | --- | | |
| Y | US-A-2 549 417  (BROWN) <br> * Colonne 1, lignes  42-44;  figures * | 1,9 | |
| | --- | | |
| A | GB-A-1 256 522   (IMS) <br> * Figures 2,3 * | 11 | |
| | --- | | |
| A | FR-A-2 003 286  (MIN-I-MIX) <br> * Page 3, lignes 7-9; figure 2 * | 8 | DOMAINES TECHNIQUES RECHERCHES (Int. Cl.4) <br><br> A 61 M <br> A 61 B |
| | ----- | | |

Le présent rapport de recherche a été établi pour toutes les revendications

| Lieu de la recherche <br> LA HAYE | Date d'achèvement de la recherche <br> 09-09-1986 | Examinateur <br> VANRUNXT J.M.A. |
|---|---|---|

OEB Form 1503 03 82

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant